(19) **Europäisches Patentamt / European Patent Office / Office européen des brevets**

(11) **EP 4 421 819 A1**

(12) **EUROPEAN PATENT APPLICATION**
published in accordance with Art. 153(4) EPC

(43) Date of publication:
**28.08.2024 Bulletin 2024/35**

(21) Application number: **22883667.2**

(22) Date of filing: **21.10.2022**

(51) International Patent Classification (IPC):
**G16H 30/40** (2018.01)   **A61N 5/10** (2006.01)
**G06T 7/00** (2017.01)   **G06T 7/60** (2017.01)

(52) Cooperative Patent Classification (CPC):
**A61N 5/10; G06T 7/00; G06T 7/60; G16H 30/40**

(86) International application number:
**PCT/JP2022/039314**

(87) International publication number:
**WO 2023/068365 (27.04.2023 Gazette 2023/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.10.2021 JP 2021172103**

(71) Applicant: **University of Tsukuba**
**Ibaraki 305-8577 (JP)**

(72) Inventors:
• **TERUNUMA, Toshiyuki**
  **Tsukuba-shi, Ibaraki 305-8577 (JP)**
• **SAKAE, Takeji**
  **Tsukuba-shi, Ibaraki 305-8577 (JP)**
• **SAKURAI, Hideyuki**
  **Tsukuba-shi, Ibaraki 305-8577 (JP)**

(74) Representative: **Osterhoff, Utz**
**Bockermann Ksoll**
**Griepenstroh Osterhoff**
**Patentanwälte**
**Bergstraße 159**
**44791 Bochum (DE)**

(54) **TARGET SHAPE ESTIMATION DEVICE AND THERAPEUTIC DEVICE**

(57)    A therapeutic device (1) estimates an outer shape of a target (30) using an identifier (61) that performed learning based on a first image (23) capturing in vivo information of a subject, a second image (30) showing the target (63) in vivo, and a third image capturing the same subject using a different device from that used to capture the first image (23). In a configuration in which the shape of a target object site is learned using a DRR image processed from a CT image, the therapeutic device (1) can accurately estimate the shape of the target object site.

FIG. 3

EP 4 421 819 A1

**Description**

Technical Field

The present invention relates to a target outer shape estimation device and a therapeutic device.

Background Art

When a tumor is treated with radiation, such as with X-rays or other radiation electromagnetic waves, or proton beams or other particle beams, it is desirable to identify the outer shape of the tumor to avoid irradiating a portion other than the tumor portion of a subject (patient). Further, since the position of the target object to be treated (e.g., tumor) moves due to breathing, pulsation, or the like of the patient, to avoid irradiating a site other than the target object (normal site) with radiation, an X-ray fluoroscopic image needs to be captured in real time to track and identify the position of the target object such that the target object is irradiated with radiation only when the target object moves to the irradiation position of the radiation.

A technique for capturing the image, identifying and tracking the position of the target object, and guiding the irradiation position of the radiation, that is, a so-called object tracking technique based on image guidance generally uses an X-ray fluoroscopic image captured using X-rays of the kV order, but no limitation is intended. For example, in addition to an X-ray fluoroscopic image, an X-ray image, an ultrasonic image, a magnetic resonance imaging (MRI) image, a computed tomography (CT) image, or a positron emission tomography (PET) image, which are captured using X-rays of the MV order, can also be used. Further, instead of the fluoroscopic image, an X-ray backscattering image using backscattering may be used.

The following technique is known as a technique for identifying and tracking the target object.

Patent Document 1 (WO 2018/159775) describes a technique for tracking a tracking target such as a tumor that moves due to a patient breathing, for example. In the technique described in Patent Document 1, a bone structure digitally reconstructed radiography (DRR) image of a bone or the like and a soft tissue DRR image of a tumor or the like are separated from an image including a tracking target (e.g., a tumor), and a plurality of superimposed images are created by randomly linearly-converting the bone structure DRR image and superimposing (randomly overlaying) the converted image on the soft tissue DRR image. Then, learning is performed using deep learning based on the plurality of superimposed images. At the time of treatment, the region of the tracking target is identified based on a fluoroscopic image at the time of treatment and the learning result, and therapeutic X-rays are irradiated onto the identified region.

Citation List

Patent Document

Patent Document 1: WO 2018/159775: WO 2018/159775A1 ("0031" to "0053", FIGS. 1 to 6)

Summary of Invention

Technical Problem

Problem of Known Technique

In the technique described in Patent Document 1, learning is performed using the DRR image created from the CT image. However, at the time of treatment, the tumor is tracked based on the fluoroscopic image with a focus on real-time tracking. Therefore, a difference in image quality between the DRR image processed from the CT image and the fluoroscopic image is a problem. When the image quality of the DRR image is lower than that of the fluoroscopic image, for example, a problem may occur in which the outer shape of the tumor is distorted, a single tumor is detected as having two or more divided regions in the image, or a tumor which does not have any holes is detected as having an internal hole in the image. When learning is performed in a state in which the tumor is not accurately recognized, there is a problem in that the accuracy of tracking the tumor deteriorates.

To solve the above-described technical problem, an object of the present invention is to accurately estimate the outer shape of a target object site with a configuration in which the shape of the target object site is learned using an image processed from a CT image.

Solution to Problem

To solve the technical problem described above, a target outer shape estimation device according to an invention described in claim 1 estimates an outer shape of a target using an identifier that performed learning based on a first image capturing in vivo information of a subject, a second image showing the target in vivo, and a third image capturing the same subject using a different device from that used to capture the first image.

With respect to the target outer shape estimation device according to the invention described in claim 1, in an invention described in claim 2, the target outer shape estimation device uses the third image that is captured before a treatment day or immediately before treatment.

With respect to the target outer shape estimation device according to the invention described in claim 1, in an invention described in claim 3, the identifier performs learning, on the target of the same subject, using a loss function that identifies a similarity among a plurality of the outer shapes of the target obtained using different methods.

With respect to the target outer shape estimation device according to the invention described in claim 3, in an invention described in claim 4, the loss function is based on a graphic similarity of the outer shapes of the target.

With respect to the target outer shape estimation device according to the invention described in claim 4, in an invention described in claim 5, the loss function is derived from a number of regions of the target and a number of internal holes of the target with respect to a shape of the target derived using the third image and the identifier.

With respect to the target outer shape estimation device according to the invention described in claim 4, in an invention described in claim 6, the loss function is derived from a number of regions of the target and a number of internal holes of the target with respect to a shape of the target derived using the first image and the identifier.

With respect to the target outer shape estimation device according to the invention described in claim 3, in an invention described in claim 7, a similar image similar to the third image is obtained from a database storing the first image, and the loss function is based on a degree of similarity between the outer shape of the target derived using the identifier and the similar image, and a similar target image identifying the outer shape of the target in the similar image.

With respect to the target outer shape estimation device according to the invention described in claim 1, in an invention described in claim 8, the identifier includes a generator configured to generate information of the outer shape of the target from the first image or the third image, and a discriminator configured to perform discrimination based on the outer shape of the target generated by the generator based on the first image, and to perform discrimination based on the outer shape of the target identified in advance and the outer shape of the target generated by the generator based on the third image, and the identifier performs learning based on a discrimination result of the discriminator.

With respect to the target outer shape estimation device according to the invention described in claim 1, in an invention described in claim 9, the first image or the third image is directly input to the identifier.

With respect to the target outer shape estimation device according to the invention described in claim 8 or 9, in an invention described in claim 10, the loss function is provided in which a loss increases as a difference between an area of the regions of the target and a predetermined value becomes larger.

With respect to the target outer shape estimation device according to the invention described in claim 8 or 9, in an invention described in claim 11, the loss function is provided in which a loss increases as a difference between an aspect ratio of the regions of the target and a predetermined value becomes larger.

With respect to the target outer shape estimation device according to the invention described in claim 8 or 9, in an invention described in claim 12, the loss function is provided in which a loss increases as a difference between a diagonal length of the regions of the target and a predetermined value becomes larger.

With respect to the target outer shape estimation device according to the invention described in claim 1, in an invention described in claim 13, each of the images is one of an X-ray image, a magnetic resonance imaging image, an ultrasonic inspection image, a positron emission tomography image, a body surface shape image, and a photoacoustic imaging image, or a combination thereof.

To solve the technical problem described above, a therapeutic device according to an invention described in claim 14 includes the target outer shape estimation device described in claim 1, and an irradiation unit configured to irradiate radiation for treatment based on the outer shape of the target estimated by the target outer shape estimation device.

Advantageous Effects of Invention

According to the inventions described in claims 1 and 14, with the configuration in which the shape of a target object site is learned using an image processed from a CT image, the shape of the target object site can be accurately estimated. According to the invention described in claim 2, learning can be deepened by using the image before the treatment day or immediately before the treatment, and the shape of the target object site can thus be accurately estimated. According to the invention described in claim 3, learning can be performed while identifying the similarity of the outer shape of the target using the loss function.

According to the invention described in claim 4, learning can be performed on the graphic similarity of the outer shape of the target using the loss function, and the shape of the target object site can thus be accurately estimated.

According to the invention described in claim 5, the shape of the target object site can be estimated by performing learning using the loss function according to the number of regions and the number of internal holes in the shape of the target derived from the third image.

According to the invention described in claim 6, the shape of the target object site can be estimated by performing learning using the loss function according to the number of regions and the number of internal holes in the shape of the target derived from the first image.

According to the invention described in claim 7, the shape of the target object site can be estimated by performing learning using the loss function derived by using the similar image similar to the third image.

According to the invention described in claim 8, the shape of the target object site can be estimated by performing learning by using the identifier including the generator and the discriminator.

According to the invention described in claim 9, the shape of the target object site can be estimated by performing learning by using the identifier to which the first image or the third image is directly input.

According to the invention described in claim 10, it can be expected that the shape of the target object site is more accurately estimated as compared with a case in which the loss function according to the area of the region of the target is not used.

According to the invention described in claim 11, it can be expected that the shape of the target object site is more accurately estimated as compared with a case in which the loss function according to the aspect ratio of the region of the target is not used.

According to the invention described in claim 12, it can be expected that the shape of the target object site is more accurately estimated as compared with a case in which the loss function according to the diagonal length of the region of the target is not used.

According to the invention described in claim 13, image capturing can be performed using one of the X-ray image, the magnetic resonance imaging image, the ultrasonic inspection image, the positron emission tomography image, the body surface shape image, and the photoacoustic imaging image, or the combination thereof, and the captured image can be utilized for treatment by X-rays or particle beams.

Brief Description of Drawings

FIG. 1 is an explanatory diagram illustrating a radiotherapy device to which a tumor outer shape estimation device according to Example 1 of the present invention is applied.

FIG. 2 is a block diagram illustrating each function of a control unit of the radiotherapy device according to Example 1.

FIG. 3 is an explanatory diagram illustrating an example of processing in the control unit according to Example 1.

FIGS. 4 are explanatory diagrams describing a relationship between the number of target regions and the number of holes. FIG. 4A is an explanatory diagram describing a case in which both the number of regions and the number of holes are 0. FIG. 4B is an explanatory diagram describing a case in which the number of regions is 1 and the number of holes is 0. FIG. 4C is an explanatory diagram describing a case in which the number of regions is 3 and the number of holes is 0. FIG. 4D is an explanatory diagram describing a case in which the number of regions is 1 and the number of holes is 1.

FIGS. 5 are explanatory diagrams describing an effect of Example 1. FIG. 5A is an explanatory diagram describing a case in which topology coefficient is not used, and FIG. 5B is an explanatory diagram describing a case in which the topology coefficient is used.

FIG. 6 is an explanatory diagram illustrating Example 2 and corresponds to FIG. 3 of Example 1.

FIG. 7 is an explanatory diagram illustrating Example 4 and corresponds to FIG. 3 of Example 1.

Description of Embodiments

Next, examples, which are specific examples of embodiments of the present invention, will be described with reference to the accompanying drawings, but the present invention is not limited to the following examples.

Note that, in the following description using the drawings, members other than members necessary for the description are appropriately omitted for ease of understanding.

Example 1

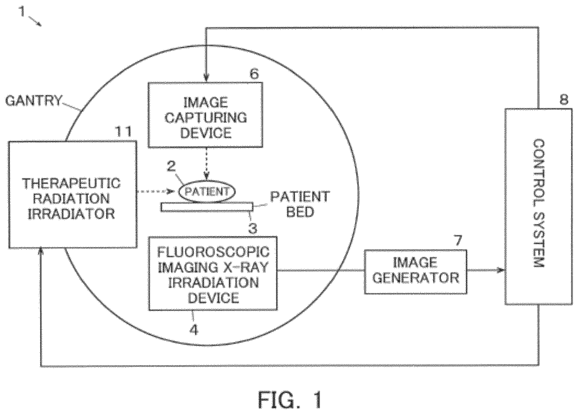

FIG. 1 is an explanatory diagram illustrating a radiotherapy device to which a tumor outer shape estimation device according to Example 1 of the present invention is applied.

In FIG. 1, a radiotherapy device (an example of a therapeutic device) 1 to which a target tracking device according to Example 1 of the present invention is applied includes a bed 3 on which a patient 2 who is a treatment subject lies. A fluoroscopic imaging X-ray irradiation device 4 is disposed below the bed 3.

An image capturing device (imaging capturing unit) 6 is disposed on the opposite side of each of the X-ray irradiation devices 4 with the patient 2 located between the image capturing unit 6 and the X-ray irradiation devices 4. The image capturing device 6 receives X-rays transmitted through the patient, and captures an X-ray fluoroscopic image. The image captured by the image capturing device 6 is converted into an electric signal by an image generator 7, and is input to a control system 8.

Further, a therapeutic radiation irradiator 11 is disposed above the bed 3. The therapeutic radiation irradiator 11 is configured such that a control signal can be input thereto from the control system 8. The therapeutic radiation irradiator 11 is configured to be able to irradiate a preset position (an affected area of the patient 2) with X-rays, as an example of therapeutic radiation, in response to input of the control signal. Note that, in Example 1, in the therapeutic radiation irradiator 11, a multi-leaf collimator (MLC, not illustrated) is installed outside an X-ray source. The MLC is an example of a diaphragm unit that can adjust an X-ray passing region (the shape of an opening through which the X-rays pass) in accordance with the outer shape of a tracking target (a tumor or the like). Note that the MLC is known as related art, and a commercially available MLC can be used. However, the configuration is not limited to the MLC, and any configuration that can adjust the X-ray passing region can be adopted.

Note that, in Example 1, X-rays having an acceleration voltage on the order of kilovolts (kV) are irradiated as the X-rays for fluoroscopic imaging, and X-rays having an acceleration voltage on the order of megavolts (MV) are irradiated as the X-rays for treatment.

Control System (Control Unit) of Example 1

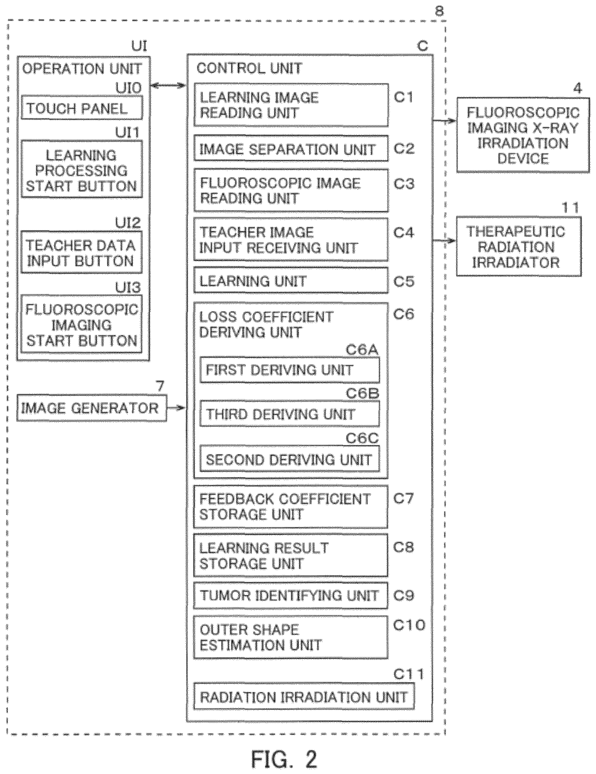

FIG. 2 is a block diagram illustrating each function of a control unit of the radiotherapy device according to Example 1.

In FIG. 2, a control unit C of the control system 8 includes an input/output interface I/O that performs inputs and outputs of signals from and to the outside, and the like. Further, the control unit C includes a read only memory (ROM) that stores a program, information, and the like for performing necessary processing. Further, the control unit C also includes a random access memory (RAM) for temporarily storing necessary data. Further, the control unit C includes a central processing unit (CPU) that performs processing in accordance with the program stored in the ROM or the like. Therefore, the control unit C according to Example 1 is constituted by a small-sized information processing device, that is, a so-called microcomputer. Thus, the control unit C can realize various functions by executing the program stored in the ROM or the like.

Signal Output Elements Connected to Control Unit C

Output signals from signal output elements such as an operation unit UI, the image generator 7, and a sensor (not illustrated) are input to the control unit C.

The operation unit (user interface) UI includes a touch panel UI0 which is an example of a display unit and an example of an input unit. Further, the operation unit UI also includes various input members such as a learning processing start button UI1, a teacher data input button UI2, and a fluoroscopic imaging start button UI3.

The image generator 7 inputs the image captured by the image capturing device 6 to the control unit C.

Controlled Elements Connected to Control Unit C

The control unit C is connected to the fluoroscopic imaging X-ray irradiation device 4, the therapeutic radiation irradiator 11, and other control elements (not illustrated). The control unit C outputs control signals to the fluoroscopic imaging X-ray irradiation device 4, the therapeutic radiation irradiator 11, and the like.

The fluoroscopic imaging X-ray irradiation device 4 irradiates the patient 2 with X-rays for capturing an X-ray fluoroscopic image, at the time of learning or treatment.

The therapeutic radiation irradiator 11 irradiates the patient 2 with therapeutic radiation (X-rays) at the time of treatment.

Functions of Control Unit C

The control unit C has a function of performing processing in accordance with the input signal from the signal output element, and outputting the control signal to each of the control elements. That is, the control unit C has the following functions.

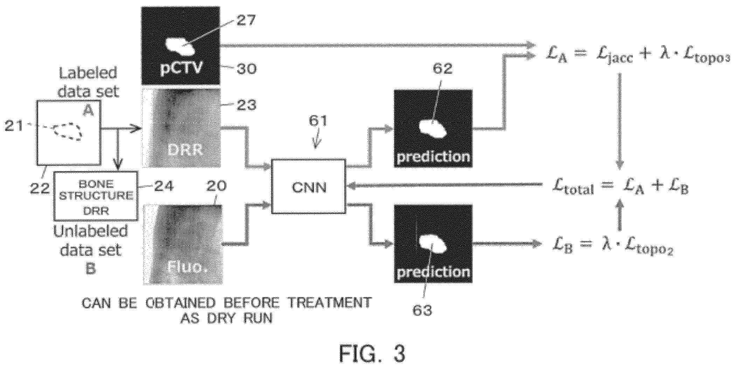

FIG. 3 is an explanatory diagram describing an example of processing in the control unit according to Example 1.

C1: Learning image reading unit (first image capturing unit)

A learning image reading unit C1 reads (reads in) an image input from the image generator 7. The learning image reading unit C1 according to Example 1 reads the image input from the image generator 7 when an input is performed on the learning processing start button UI1. In Example 1, after the input on the learning processing start button UI1 is started, reading of an X-ray CT image (first fluoroscopic image) is performed during a preset learning period. In Example 1, the learning processing is not performed in real time along with the image capturing of the X-ray CT image. However, if the processing speed is improved due to an increased speed of the CPU or the like and the processing can be performed in real time, the learning processing can be performed in real time.

C2: Image separation unit

An image separation unit C2 separates the X-ray CT image into a tracking object portion image 23 (e.g., a soft tissue digitally reconstructed radiography (DRR) image) including a tracking object image region (region of the target) 21, and a separated background image 24 (e.g., a bone structure DRR image (bone image) including an image region of a bone structure) not including the tracking object image region 21, based on an original image 22 including three-dimensional information for learning, which includes the tracking object image region 21, and extracts the tracking object portion image 23 and the separated background image 24. The image separation unit C2 according to Example 1 separates the CT image into the tracking object portion image 23 (first image, soft tissue DRR image) and the separated background image 24 (bone structure DRR image), based on a CT value which is contrast information of the CT image. In Example 1, as an example, a region having a CT value of 200 or more is used as the bone structure DRR image to form the separated background image 24, and a region having a CT value of less than 200 is used as the soft tissue DRR image to form the tracking object portion image 23.

Note that, as an example, Example 1 is an example in which a tumor (tracking object) developed in a lung is detected, that is, in which the target of the treatment is captured in the tracking object portion image 23 as the soft tissue DRR image. However, for example, when the tracking object is an abnormal portion of a bone or the like, the bone structure DRR image is selected as the tracking object portion image 23, and the soft tissue DRR image is selected as the separated background image 24. In this manner, the selection of the tracking object portion image and the background image (non-tracking object image) is appropriately performed in accordance with the tracking object (target) and the background image including an obstacle.

C3: Fluoroscopic image reading unit (second image capturing unit)

A fluoroscopic image reading unit C3 reads (reads in) an image input from the image generator 7. The fluoroscopic image reading unit C3 according to Example 1 reads (obtains) the image input from the image generator 7 when an input to the fluoroscopic imaging start button UI3 is performed. In Example 1, the X-rays irradiated from the fluoroscopic imaging X-ray irradiation device 4 are transmitted through the patient 2, and an X-ray fluoroscopic image 20 (third image), which is an image captured by each of the image capturing devices 6, is read.

C4: Teacher image input receiving unit (target image storage unit)

A teacher image input receiving unit C4 receives an input of a teacher image (second image) 30 including a teacher image region 27 as an example of an image for teaching a target to be tracked, in response to an input to the touch panel UI0 or an input to the teacher data input button UI2. Note that, in Example 1, a configuration is adopted in which the original image (CT image) 22 for learning is displayed on the touch panel UI0, and a doctor can determine the teacher image region 27 by performing input on the screen and thus surrounding an image region of the tracking object, that is, a target object of the treatment.

C5: Learning unit (identifier creation unit)

A learning unit C5 learns at least one of region information and position information of the tracking object image region 21 in the image, based on a plurality of the X-ray fluoroscopic images 20 and the tracking object portion image (soft tissue DRR image) 23, and creates an identifier 61. In Example 1, both the region and the position of the tracking object image region 21 are learned. In Example 1, the X-ray fluoroscopic image 20 and the DRR image 23 are directly input to the identifier 61.

Further, in Example 1, the position of the center of gravity of the tracking object image region 21 is set as the position of the tracking object image region 21. However, the position may be changed to an arbitrary position such as the upper end, the lower end, the right end, or the left end of the region in accordance with a design, specifications, or the like. The learning unit C5 can adopt any known configuration of related art, but it is preferable to use so-called deep learning (a neural network having a multilayer structure), and it is particularly preferable to use a convolutional neural network (CNN). In Example 1, Caffe is used as an example of the deep learning, but the configuration is not limited thereto, and any learning method (framework, algorithm, software) can be employed.

C6: Loss coefficient deriving unit

A loss coefficient deriving unit C6 derives (calculates) a loss coefficient from the identifier (CNN) 61 derived by the learning unit C5. The loss coefficient deriving unit C6 according to Example 1 includes a first deriving unit C6A, a third deriving unit C6B, and a second deriving unit C6C.

C6A: First deriving unit

The first deriving unit C6A derives an outer shape of a target (an estimated target image) 62 using the soft tissue DRR image (first image) 23 and the identifier 61 once derived by the learning unit C5, and derives a Jaccard coefficient (first loss coefficient) $L_{jacc}$ that indicates a degree of similarity between the derived outer shape of the target 62 and the teacher image region 27 (outer shape of the target) of the teacher image 30 (target image). Note that the Jaccard coefficient $L_{jacc}$ is a known coefficient or function indicating a degree of similarity between two sets, and thus a detailed description thereof will be omitted. Further, in Example 1, an example is described in which the Jaccard coefficient is used as an example of the first loss function indicating the degree of similarity, but the configuration is not limited thereto. For example, as another function indicating the degree of similarity, a Dice coefficient, a Simpson coefficient (overlap coefficient), or the like can be used.

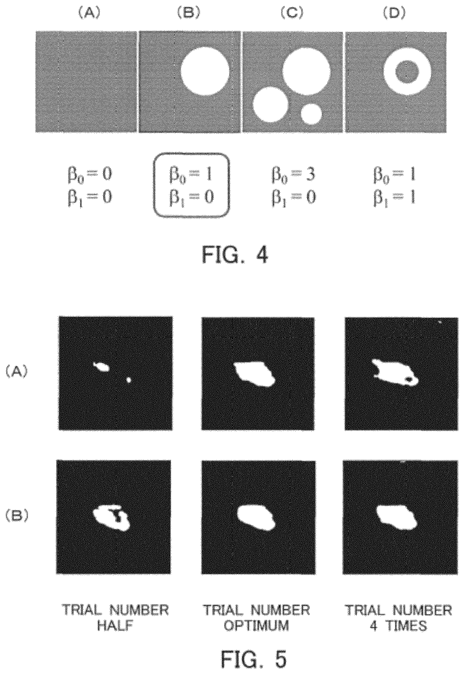

FIGS. 4 are explanatory diagrams describing a relationship between the number of target regions and the number of holes of the target. FIG. 4A is an explanatory diagram describing a case in which both the number of regions and the number of holes are 0. FIG. 4B is an explanatory diagram describing a case in which the number of regions is 1 and the number of holes is 0. FIG. 4C is an explanatory diagram describing a case in which the number of regions is 3 and the number of holes is 0. FIG. 4D is an explanatory diagram describing a case in which the number of regions is 1 and the number of holes is 1.

C6B: Third deriving unit

The third deriving unit C6B derives a third topology coefficient (third loss function) $L_{topo3}$ based on a number $\beta_0$ of target regions and a number $\beta_1$ of internal holes in the shape of the target (outer shape of the target 62) derived using the soft tissue DRR image (first fluoroscopic image) 23 and the identifier 61 once derived by the learning unit C5. When the derived outer shape of the target 62 is as illustrated in FIG 4A, $\beta_0 = 0$ and $\beta_1 = 0$. When the derived outer shape of the target 62 is as illustrated in FIG 4B, $\beta_0 = 1$ and $\beta_1 = 0$. When the derived outer shape of the target 62 is as illustrated in FIG 4C, $\beta_0 = 3$ and $\beta_1 = 0$. When the derived outer shape of the target 62 is as illustrated in FIG 4D, $\beta_0 = 1$ and $\beta_1 = 1$. Note that, in reality, in a normal radiation treatment, the target has one region, and the region should not be divided, or the region should not have a hole therein. That is, in reality, $\beta_0 = 1$ and $\beta_1 = 0$ should be satisfied. Therefore, with respect to cases other than the case in which $\beta_0 = 1$ and $\beta_1 = 0$, a penalty, that is, the topology coefficient $L_{topo3}$, which is a loss coefficient indicating an incorrect solution, is defined. In Example 1, as an example, the topology coefficient $L_{topo3}$ is defined by the following equation (1).

$$L_{topo3} = (1/nb) \; \Sigma_{i=1}^{nb}(1 - \beta_{0,i} - \beta_{1,i})^2 \cdots \text{Equation (1)}$$

Note that, in Equation (1), nb is a batch number and indicates the number of images processed at a time. In other words, in Equation (1), even when the number of processed sheets increases or decreases, the topology coefficient

$L_{topo3}$ is standardized (normalized) by using the multiplication by "(1/nb)".

Equation (1) according to Example 1 is a function (loss function) in which the topology coefficient $L_{tpo3}$ is minimized when $\beta_0 = 1$ and $\beta_1 = 0$, which is a correct solution, and the value thereof is increased when the solution is incorrect.

Further, in Example 1, the topology coefficient $L_{topo3}$ is expressed by the function defined by Equation (1) as an example, but the configuration is not limited thereto. For example, Equation (1) is made to produce a positive number by squaring, but instead of this, Equation (2) that is a function using an absolute value without squaring may be used.

$$L_{topo3} = (1/nb)\Sigma^{nb}_{i=1} \mid 1 - \beta_{0,i} - \beta_{1,i} \mid \cdots \text{Equation (2)}$$

In addition, in a case in which two separate tumors are present, it is also possible to define the topology coefficient and thus simultaneously satisfy the following equations (3) and (4), as a loss function that determines solutions other than $\beta_0 = 2$ and $\beta_1 = 0$, which is a correct solution, as being incorrect.

$$L_{topo3} = (1/nb)\Sigma^{nb}_{i=1}(2 - \beta_{0,i} - \beta_{1,i})^2 \cdots \text{Equation (3)},$$

and

$$\Sigma^{nb}_{i=1}\beta_{1,i} = 0 \cdots \text{Equation (4)}$$

Similarly, when the number of regions is three or more, the topology coefficient is set in accordance with the correct solution.

Further, the topology coefficient is a two dimensional topology coefficient in Example 1, but when it is three-dimensional, it is also possible to use a topology coefficient to which a parameter such as $\beta_2$ is added.

C6C: Second deriving unit

The second deriving unit C6C derives a second topology coefficient (second loss function) $L_{topo2}$ based on the number $\beta_0$ of target regions and the number $\beta_1$ of internal holes in the shape of the target (outer shape of the target (estimated target image) 63) derived using the X-ray fluoroscopic image 20 and the identifier 61 once derived by the learning unit C5. Note that the second topology coefficient $L_{topo2}$ has the same definition as that of the third topology coefficient $L_{topo3}$ except that the shape of the target object is different. Thus, a detailed description of the second topology coefficient $L_{topo2}$ is omitted.

C7: Feedback coefficient storage unit

A feedback coefficient storage unit C7 stores a coefficient $\lambda$ used when feeding back the topology coefficients $L_{topo2}$ and $L_{topo3}$. In Example 1, as an example, the feedback coefficient $\lambda$ is set to 0.01. Note that the feedback coefficient $\lambda$ can be arbitrarily changed according to the design, specifications, required learning accuracy, learning time, and the like.

The learning unit C5 according to Example 1 further learns the identifier 61 based on the first loss function $L_{jacc}$, the second loss function $L_{topo2}$, and the third loss function $L_{topo3}$.

C8: Learning result storage unit

A learning result storage unit C8 stores a learning result of the learning unit C5. In other words, the CNN optimized by relearning is stored as the final identifier 61.

C9: Tumor identifying unit (target identifying unit)

A tumor identifying unit C9 identifies the outer shape of a tumor, which is the target, based on the captured image when performing treatment. Note that, as an identification method, it is possible to employ a known image analysis technique of related art, it is possible to perform discrimination by reading in a plurality of images of the tumor and performing learning, or it is also possible to use the technique described in Patent Document 1. Note that the "outer shape" of the target is not limited to the outer shape of the tumor itself (= the boundary between the normal site and the tumor), but there may be a case in which the outer shape of the target is set inside or outside the tumor based on a doctor's judgment or the like. That is, the "outer shape" may be a region specified by a user. Therefore, the "target" is not limited to the tumor, but may be a region that the user wants to track.

C10: Outer shape estimation unit

An outer shape estimation unit C10 estimates the outer shape of the tumor based on the X-ray fluoroscopic image 20 captured immediately before performing the treatment and the identifier 61, and outputs the estimation result.

C11: Radiation irradiation unit

A radiation irradiation unit C11 controls the therapeutic irradiator 11 to irradiate the therapeutic X-rays when the region and the position of the tumor estimated by the outer shape estimation unit C10 are included in an irradiation range of the therapeutic X-rays. Note that the radiation irradiation unit C11 according to Example 1 controls the MLC in accordance with the region (outer shape) of the tumor estimated by the outer shape estimation unit C10, and thus adjusts an irradiation region (irradiation field) of the therapeutic X-rays to be the outer shape of the tumor. Note that when the therapeutic X-rays are irradiated, the radiation irradiation unit C11 controls the MLC in real time, in accordance with the output of the estimation result of the outer shape of the tumor that changes over time.

Note that a target outer shape estimation device of Example 1 is constituted by the fluoroscopic imaging X-ray irradiation device 4, the image capturing devices 6, the image generator 7, the control system 8, and each of the units C1 to C11.

Effect of Example 1

In a radiotherapy device 1 according to Example 1 having the above-described configuration, learning is performed using the X-ray fluoroscopic image 20 and the soft tissue DRR image 23. In other words, the learning is performed in a state in which the DRR image 23 and the X-ray fluoroscopic image 20 are mixed, so to speak. Thus, even when there is a difference in image quality between the DRR image 23 and the X-ray fluoroscopic image 20, the learning is performed in a state in which both the images are mixed. Therefore, it is possible to improve the image quality as compared with the related art in which the learning is performed using only the DRR image. Thus, it is possible to accurately estimate the outer shape of the tumor. Therefore, it is possible to accurately irradiate the tumor or the affected area with X-rays during treatment.

In particular, a technique of performing learning using only the supervised DRR image 23 and a technique of performing learning using only the unsupervised X-ray fluoroscopic image 20 have been available individually, but learning of the supervised image and the unsupervised image in the mixed state using topology, as in Example 1, has not been performed in the related art.

On the other hand, in Example 1, the accuracy is improved by performing the learning by mixing the two images (the DRR image 23 and the X-ray fluoroscopic image 20), which are different in modality (classification, form), using the topology. In particular, in Example 1, the tracking object portion image 23 and the teacher image 30 are paired images, and in this case, supervised learning is possible. On the other hand, the X-ray fluoroscopic image 20 is in a state of not having a teacher image (in a non-paired image state), but even in this case, learning is possible while including the X-ray fluoroscopic image 20.

Note that, for the X-ray fluoroscopic image 20, it is difficult to provide a perfectly correct image (teacher image) because the contrast between a malignant tumor and the normal site is small on the image, and further, it is difficult to perform learning in a supervised state because the shape and size of the tumor may change in the course of daily progression of symptoms.

FIGS. 5 are explanatory diagrams describing the effect of Example 1. FIG. 5A is an explanatory diagram describing a case in which the topology coefficient is not used, and FIG. 5B is an explanatory diagram describing a case in which the topology coefficient is used.

In particular, in Example 1, a degree of similarity $L_A$ (= $L_{jacc}$ + $\lambda \cdot L_{topo3}$) between the DRR image 23 and the outer shape of the target 62 derived from the identifier 61 is derived. Further, a degree of similarity $L_B$ (= $\lambda \cdot L_{topo2}$) between the X-ray fluoroscopic image 20 and an outer shape of a target 63 derived from the identifier 61 is derived. Then, a sum $L_{total}$ of the two degrees of similarity is fed back and relearned. When only the known Jaccard coefficient of the related art is used, as illustrated in FIG. 5A, a problem occurs in which the number of regions is two or more (when the learning time is half the optimum time and is too short), or in which a hole is present inside the region (when the learning time is four times the optimum time and is too long). On the other hand, in Example 1, as illustrated in FIG. 5B, the state is stabilized after a certain period of time or more elapses.

In particular, the X-ray CT image is captured on the day of diagnosis or the day of planning a treatment plan, and it is not frequently captured until the day of treatment due to the burden on the patient such as exposure to radiation, but the X-ray fluoroscopic images are typically captured a plurality of times before the day of treatment. Therefore, with respect to the DRR image 23 based on the X-ray CT image, the first captured image may be used, and subsequent changes in the shape of the tumor can be repeatedly relearned using the second topology coefficient $L_{topo2}$ based on the X-ray fluoroscopic images 20. In other words, while taking advantage of the fact that the X-ray fluoroscopic image 20 is a non-paired image and does not incur the cost of creating the teacher data, the X-ray fluoroscopic image 20 can be used for learning immediately after the image is obtained. In this manner, the accuracy can also be improved by performing the relearning using the latest X-ray fluoroscopic image.

Further, in Example 1, since the learning is performed using the captured image of the patient 2 to whom the treatment is performed, the accuracy is improved compared to a case in which a captured image of a third person is used.

Example 2

Next, Example 2 of the present invention will be described. In the description of Example 2, constituent elements corresponding to the constituent elements of Example 1 are denoted by the same reference signs, and detailed description thereof will be omitted.

This example is different from Example 1 in the following points, but is configured similarly to Example 1 in other points.

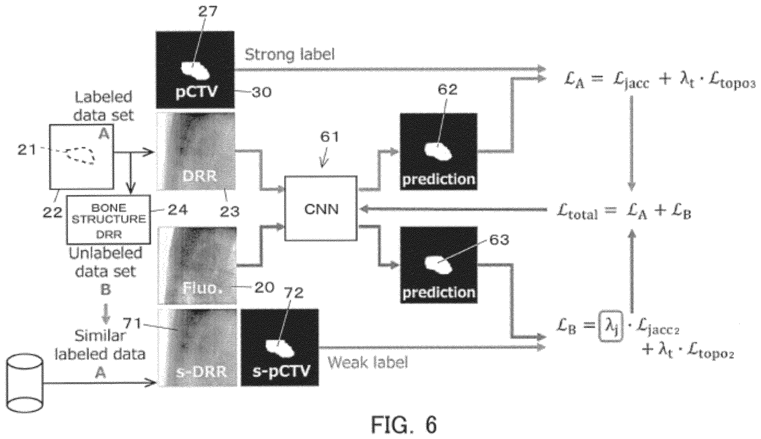

FIG. 6 is an explanatory diagram describing Example 2, and corresponds to FIG. 3 of Example 1.

Although only the second topology coefficient $L_{topo2}$ is calculated for the X-ray fluoroscopic image 20 in Example 1, the Jaccard coefficient is also derived in Example 2 as illustrated in FIG. 6. In Example 2, a DRR image (similar DRR image 71) similar to the X-ray fluoroscopic image 20 is searched for and obtained from a database of the DRR image of the subject. A known image analysis technique can be used to search for the similar image. Then, a second Jaccard coefficient (fourth loss function) $L_{jacc2}$, which is a degree of similarity between an outer shape of a target (similar target image) 72 associated with the retrieved similar DRR image 71 and the outer shape of the target 63, is derived.

The learning unit C5 according to Example 2 further learns the identifier 61 based on the first loss function $L_{jacc}$, the second loss function $L_{topo2}$, the third loss function $L_{topo3}$, and the fourth loss function $L_{jacc2}$. Specifically, relearning is performed using a feedback value $L_{total}$ (= $L_{jacc} + \lambda \cdot L_{topo3} + \lambda j \cdot L_{jacc2} + \lambda \cdot L_{topo2}$).

Note that the similar target image 72 according to Example 2 is not strictly accurate teacher data, but rather "slightly inaccurate teacher data". Thus, in Example 2, the second Jaccard coefficient $L_{jacc2}$ is not fed back as it is, but multiplied by a feedback coefficient $\lambda j$ (< 1) and then fed back. Although the feedback coefficient $\lambda j$ according to Example 2 is set to $\lambda j = 0.3$ as an example, a specific numerical value can be appropriately changed in accordance with the design, specifications, or the like.

Effect of Example 2

In the radiotherapy device 1 according to Example 2 having the above-described configuration, the X-ray fluoroscopic image 20 is also fed back using the second Jaccard coefficient $L_{jacc2}$. In other words, in Example 2, the similar DRR image 71 is searched for the X-ray fluoroscopic image 20 that does not have the teacher image, and the similar target image 72 associated with the similar DRR image 71 is used. As a result, the learning is somewhat forcibly performed in a state (a state with the slightly inaccurate teacher data) close to a state with a teacher (with the correct image). Therefore, it can be expected that the outer shape of the tumor is estimated more accurately than in Example 1.

Example 3

Next, Example 3 of the present invention will be described. In the description of Example 3, constituent elements corresponding to the constituent elements of Example 1 are denoted by the same reference signs, and detailed description thereof will be omitted.

This example is different from Example 1 in the following points, but is configured similarly to Example 1 in other points.

In Example 1, the topological coefficients $L_{topo2}$ and $L_{topo3}$, which are the loss functions, are used to consider the number of regions and the number of holes. However, in Example 3, loss functions based on the area, the aspect ratio, and the diagonal length of the target are also used.

An area coefficient $L_{area}$, which is a loss function based on the area, is calculated based on an area $S_{area}$ of the target and a predetermined assumed value $S_{true1}$ using the following equation (5), and thus the loss increases as a difference between the area $S_{area}$ of the target and the assumed value $S_{true1}$ becomes larger.

$$L_{area} = (1/nb)\Sigma^{nb}_{i=1}(S_{area} - S_{true1})^2 \cdots \text{Equation (5)}$$

Similarly, an aspect ratio coefficient $L_{asp}$, which is a loss function based on the aspect ratio, is calculated based on an aspect ratio $S_{asp}$ of the target and a predetermined assumed value $S_{true2}$ using the following equation (6), and thus the loss increases as a difference between the aspect ratio $S_{asp}$ of the target and the assumed value $S_{true2}$ becomes larger.

$$L_{asp} = (1/nb)\Sigma^{nb}_{i=1}(S_{sp} - S_{true2})^2 \cdots \text{Equation (6)}$$

Further, a diagonal coefficient $L_{diag}$, which is a loss function based on the diagonal length, is calculated based on a diagonal length $S_{diag}$ of the target and a predetermined assumed value $S_{true3}$ using the following equation (7), and thus the loss increases as a difference between the diagonal length $S_{diag}$ of the target and the assumed value $S_{true3}$ becomes larger.

$$L_{diag} = (1/nb)\Sigma^{nb}_{i=1}(S_{diag} - S_{true3})^2 \cdots \text{Equation (7)}$$

In Example 3, the topology coefficients $L_{topo2}$, $L_{area1}$, $L_{asp1}$, and $L_{diag}$, are derived for the outer shape of the target 62 based on the DRR image 23, and the topology coefficients $L_{topo3}$, $L_{area2}$, $L_{asp2}$, and $L_{diag2}$ are derived for the outer shape of the target 63 based on the X-ray fluoroscopic image 20. Then, the degree of similarity $L_A$ (= $L_{jacc}$ + $\lambda \cdot L_{topo2}$ + $\lambda_{ar} \cdot L_{area1}$ + $\lambda_{as} \cdot L_{asp1}$ + $\lambda_d \cdot L_{diag1}$), and the degree of similarity $L_B$ (= $\lambda \cdot L_{topo3}$ + $\lambda_{ar} \cdot L_{area2}$ + $\lambda_{as} \cdot L_{asp2}$ + $\lambda_d \cdot L_{diag2}$) are derived, respectively. Here, $\lambda_{ar}$ is a feedback coefficient with respect to the area coefficient $L_{area}$. $\lambda_{as}$ is a feedback coefficient with respect to the aspect ratio coefficient $L_{asp}$, and $\lambda_d$ is a feedback coefficient with respect to the diagonal length coefficient $L_{diag}$. Note that the second Jaccard coefficient $L_{jacc2}$ according to Example 2 may be applied in place of the degree of similarity $L_B$.

Effect of Example 3

In the radiotherapy device 1 according to Example 3 having the above-described configuration, when the area $S_{area}$ of the outer shape of the target 62, 63 of the tumor, which is estimated using the identifier 61, is extremely larger or extremely smaller than the assumed area $S_{true1}$, a penalty is applied from the calculation of the area coefficient $L_{area}$, and the estimation result is likely to be incorrect.

Further, when the aspect ratio $S_{asp}$ of the outer shape of the target 62, 63 of the tumor, which is estimated using the identifier 61, is extremely larger or extremely smaller than the assumed aspect ratio $S_{true2}$, a penalty is applied from the calculation of the aspect ratio coefficient $L_{asp}$, and the estimation result is likely to be incorrect.

Furthermore, when the diagonal length $S_{diag}$ of the outer shape of the target 62, 63 of the tumor, which is estimated using the identifier 61, is extremely larger or extremely smaller than the assumed aspect ratio $S_{true3}$, a penalty is applied from the calculation of the diagonal coefficient $L_{diag}$, and the estimation result is likely to be incorrect.

Thus, in Example 3, it can be expected that the outer shape of the tumor is estimated more accurately than in Example 1.

Example 4

Next, Example 4 of the present invention will be described. In the description of Example 4, constituent elements corresponding to the constituent elements of Example 1 are denoted by the same reference signs, and detailed description thereof will be omitted.

This example is different from Example 1 in the following points, but is configured similarly to Example 1 in other points.

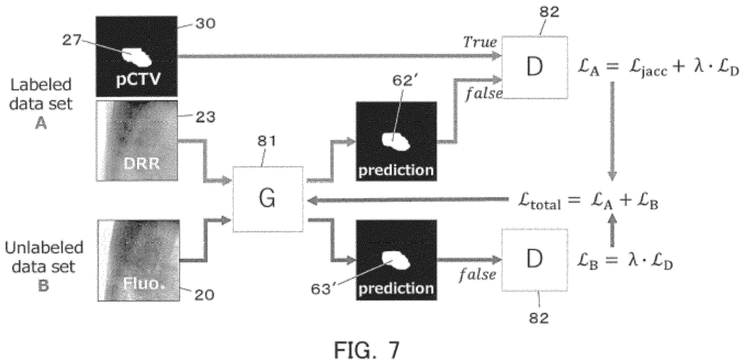

FIG. 7 is an explanatory diagram describing Example 4 and corresponds to FIG. 3 of Example 1.

In FIG. 7, the radiotherapy device 1 according to Example 4 includes a generator 81 that generates an outer shape of a target 62' from the DRR image 23 (an example of the first image in Example 4) and that generates an outer shape of a target 63' from the X-ray fluoroscopic image 20 (an example of the third image in Example 4), instead of the identifier 61 according to Example 1. The generator 81 according to Example 4 generates the images 62' and 63' by applying random numbers and noise to the input images 20 and 23, respectively. Therefore, the images 62' and 63' generated by the generator 81 are so-called false images (images estimated to be false).

Further, the radiotherapy device 1 according to Example 4 includes a discriminator 82. The discriminator 82 according to Example 4 outputs a discrimination result (discrimination result of true or false) from the image (false image) 62' generated from the DRR image 23 by the generator 81 and the teacher image (true image) 30, or outputs a discrimination result (true or false) from the image (false image) 63' generated from the X-ray fluoroscopic image 20 by the generator 81. The generator 81 is learned from loss functions $L_{jacc}$ and $L_D$ derived based on the teacher image 30 and the image 62' or on the image 63'. $L_{jacc}$ is calculated from the degree of similarity of the outer shape of the target between the teacher image 30 and the image 62', and $L_D$ is calculated from the outer shapes of the target of the images 62' and 63'.

Therefore, in Example 1, a generative adversarial network (GAN) using the generator 81 and the discriminator 82 is used as the identifiers 81 + 82.

Note that although two of the discriminators 82 are illustrated in FIG. 7, the two discriminators 82 are illustrated to explain the flow of information processing, and only one of the discriminators 82 is actually provided. This is because, in the radiotherapy device 1 according to Example 4, it is necessary to output a similar shape at the time of training and inference, and thus the learning is performed while the discriminator 82 determines if the image is the similar image.

Effect of Example 4

In the radiotherapy device 1 according to Example 4 having the above-described configuration, it is possible to deepen the learning by using the generator 81 and the discriminator 82 and repeating the generation of a large number of false images by the generator 81 and the discrimination between true and false by the discriminator 82. Therefore, the accuracy is also improved in Example 4.

Note that the loss functions are not limited to those based on the topology calculation, and can be expressed by a neural network (CNN 61, the generator 81, the discriminator 82) as an identifier, while taking advantage of the fact that the outer shape of the target of the same patient has substantially the same shape regardless of the image capturing device.

Modified Examples

Although examples of the present invention have been described above in detail, the present invention is not limited to the examples described above, and various modifications and changes can be made to the examples without departing from the scope of the present invention as described in the claims. Modified examples (H01) to (H03) of the present invention will be described below as examples.

(H01) In the above-described examples, a configuration is illustrated, as an example, in which the X-ray fluoroscopic image using the X-rays of the kV order is used as the captured image, but the configuration is not limited thereto. For example, it is also possible to use an X-ray image using X-rays of the MV order (an image detected after therapeutic MV-X-rays have passed through the patient), an ultrasonic inspection image (so-called ultrasonic echo image), a magnetic resonance imaging (MRI) image, a positron emission tomography (PET) image, a photoacoustic imaging (PAI) image, an X-ray backscattering image, or the like. Further, a modification such as combining the X-ray image (kV-X rays or MV-X rays) and the MRI image or the like is also possible.

In addition, it is also possible to capture an image of a body surface shape of the patient, which changes due to breathing or the like, using an image capturing unit such as a 3D camera or a distance sensor, and to estimate the position and the outer shape of a tumor using a body surface shape image, while taking advantage of the fact that the motion of breathing or the like is deeply correlated with the motion of the tumor.

(H02) In the above-described embodiments, a configuration is illustrated, as an example, in which tracking is performed without using a marker, but the configuration is not limited thereto. It is also possible to use an X-ray image or the like in which a marker is embedded.

(H03) In the above-described embodiments, a configuration is illustrated, as an example, in which the third loss function $L_{topo3}$ is used when deriving the degree of similarity $L_A$ of the outer shape of the target 62 derived from the DRR image 23 and the identifier 61, but the configuration is not limited thereto. Depending on the required accuracy, a configuration may be adopted in which the third loss function $L_{topo3}$ is not used.

Reference Signs List

1 Therapeutic device

2 Subject

20 Third image

23 First image

30 Second image

61, 81 + 82 Identifier

62, 63, 62', 63'Outer shape of target, target estimation image

71 Similar image

72 Similar target image

81 Generator

82 Discriminator

C1 First image capturing unit

C3 Second image capturing unit

C4 Target image storage unit

C5 Learning unit

C6A First deriving unit

C6B Third deriving unit

C6C Second deriving unit

C10 Outer shape estimation unit

C11 Irradiation unit

$L_{jacc}$ First loss function

$L_{jacc}$, $L_{jacc2}$, $L_{topo2}$, $L_{topo3}$, $L_{area1}$, $L_{area2}$, $L_{asp1}$, $L_{asp2}$, $L_{diag1}$, $L_{diag2}$. Loss function

$L_{jacc2}$. Fourth loss function

$L_{topo2}$, $L_{area1}$, $L_{asp1}$, $L_{diag1}$. Second loss function

$L_{topo3}$, $L_{area2}$, $L_{asp2}$, $L_{diag2}$. Third loss function

$S_{area}$. Area of region of target

$S_{asp}$ Aspect ratio of region of target

$S_{diag}$. Diagonal length of region of target

$S_{true1}$, $S_{true2}$, $S_{true3}$. Predetermined value

$\beta_0$. Number of target regions

$\beta_1$ Number of internal holes

**Claims**

1. A target outer shape estimation device configured to estimate an outer shape of a target using an identifier that performed learning based on a first image capturing in vivo information of a subject, a second image showing the target in vivo, and a third image capturing the same subject using a different device from that used to capture the first image.

2. The target outer shape estimation device according to claim 1, wherein
the target outer shape estimation device uses the third image that is captured before a treatment day or immediately before treatment.

3. The target outer shape estimation device according to claim 1, wherein
the identifier performs learning, on the target of the same subject, using a loss function that identifies a similarity among a plurality of the outer shapes of the target obtained using different methods.

4. The target outer shape estimation device according to claim 3, wherein
the loss function is based on a graphic similarity of the outer shapes of the target.

5. The target outer shape estimation device according to claim 4, wherein
the loss function is derived from a number of regions of the target and a number of internal holes of the target with respect to a shape of the target derived using the third image and the identifier.

6. The target outer shape estimation device according to claim 4, wherein
the loss function is derived from a number of regions of the target and a number of internal holes of the target with respect to a shape of the target derived using the first image and the identifier.

7. The target outer shape estimation device according to claim 3, wherein

a similar image similar to the third image is obtained from a database storing the first image, and
the loss function is based on a degree of similarity between the outer shape of the target derived using the identifier and the similar image, and a similar target image identifying the outer shape of the target in the similar image.

8. The target outer shape estimation device according to claim 1, wherein
the identifier includes

a generator configured to generate information of the outer shape of the target from the first image or the third image, and
a discriminator configured to perform discrimination based on the outer shape of the target generated by the generator based on the first image, and to perform discrimination based on the outer shape of the target identified in advance and the outer shape of the target generated by the generator based on the third image, and
the identifier performs learning based on a discrimination result of the discriminator.

9. The target outer shape estimation device according to claim 1, wherein
the first image or the third image is directly input to the identifier.

10. The target outer shape estimation device according to claim 8 or 9, wherein
the loss function is provided in which a loss increases as a difference between an area of the regions of the target and a predetermined value becomes larger.

11. The target outer shape estimation device according to claim 8 or 9, wherein
the loss function is provided in which a loss increases as a difference between an aspect ratio of the regions of the target and a predetermined value becomes larger.

12. The target outer shape estimation device according to claim 8 or 9, wherein
the loss function is provided in which a loss increases as a difference between a diagonal length of the regions of the target and a predetermined value becomes larger.

13. The target outer shape estimation device according to claim 1, wherein
each of the images is one of an X-ray image, a magnetic resonance imaging image, an ultrasonic inspection image, a positron emission tomography image, a body surface shape image, and a photoacoustic imaging image, or a combination thereof.

14. A therapeutic device, comprising:

the target outer shape estimation device according to claim 1; and
an irradiation unit configured to irradiate radiation for treatment based on the outer shape of the target estimated by the target outer shape estimation device.

FIG. 1

FIG. 2

FIG. 3

(A)     (B)     (C)     (D)

$\beta_0 = 0$
$\beta_1 = 0$

$\beta_0 = 1$
$\beta_1 = 0$

$\beta_0 = 3$
$\beta_1 = 0$

$\beta_0 = 1$
$\beta_1 = 1$

FIG. 4

(A)

(B)

TRIAL NUMBER     TRIAL NUMBER     TRIAL NUMBER
HALF           OPTIMUM         4 TIMES

FIG. 5

FIG. 6

FIG. 7

## INTERNATIONAL SEARCH REPORT

International application No.

**PCT/JP2022/039314**

### A. CLASSIFICATION OF SUBJECT MATTER

*G16H 30/40*(2018.01)i; *A61N 5/10*(2006.01)i; *G06T 7/00*(2017.01)i; *G06T 7/60*(2017.01)i
FI:    G16H30/40; A61N5/10 M; G06T7/00 350C; G06T7/60 150S

According to International Patent Classification (IPC) or to both national classification and IPC

### B. FIELDS SEARCHED

Minimum documentation searched (classification system followed by classification symbols)

G16H30/40; A61N5/10; G06T7/00; G06T7/60

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2018/083844 A1 (SHIMADZU CORP) 11 May 2018 (2018-05-11) paragraphs [0082]-[0085], [0108]-[0113], [0141]-[0142], fig. 8, 12, 19-20 | 1-14 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 December 2022** | **27 December 2022** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 **Japan** | |
| | Telephone No. |

Form PCT/ISA/210 (second sheet) (January 2015)

INTERNATIONAL SEARCH REPORT
Information on patent family members

International application No.

**PCT/JP2022/039314**

| Patent document cited in search report | | | Publication date (day/month/year) | Patent family member(s) | | | Publication date (day/month/year) |
|---|---|---|---|---|---|---|---|
| WO | 2018/083844 | A1 | 11 May 2018 | US | 2020/0069967 | A1 | |
| | | | | paragraphs [0102]-[0105], [0128]-[0133], [0161]-[0162], fig. 8, 12, 19-20 | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018159775 A

- WO 2018159775 A1